# EUROPEAN PATENT APPLICATION

(11) **EP 1 897 584 A1**
(43) Date of publication of application: **12.03.2008**
(21) Application number: 06425621.7
(22) Date of filing: 07.09.2006
(51) Int. Cl.: A61M 25/10, A61M 25/00, A61L 29/00, A61M 29/00

(54) **Angioplasty catheter**

(71) Applicant: Parente, Manuel, 6900 Lugano (CH)
(72) Inventor: Parente, Manuel, 6900 Lugano (CH)
(74) Representative: Vinci, Marcello

(57) **Abstract**

This is a new angioplasty catheter comprising a tip portion (P), a proximal tube (C1) in flexible plastic material around which the inflatable balloon (B) is fitted, a multilayer distal tube (C2) in flexible plastic material, a metal tube (C3) for grip and connection. On the proximal tube (C1) there are two marking radio-opaque metal rings (M). It is possible to have also the proximal tube (C1) constituted by two or more layers. It is possible for the balloon (B) to have a truncated conical shape so as to operate as required in blood vessels with a decreasing internal diameter.

## Description

### FIELD OF THE INVENTION

The present patent concerns artery angioplasty treatments and in particular it concerns the dilation of the arteries by means of catheters equipped with an inflatable balloon.

### PRIOR ART

To remove or reduce a plaque that clogs an artery, angioplasty or Percutaneous Transluminal Coronary Angioplasty (PTCA) is currently practised, that is, the use of a blind catheter with a balloon enveloping the catheter itself near its inserting end. This balloon is deflated, that is, it adheres to the catheter, while it is being inserted in the artery and slipped until it reaches the position where the artery is clogged.

When the catheter is correctly positioned, that is, when its portion with the balloon is in the residual lumen of the occlusion, air or another fluid (for example water for injections) is blown into the inside of the catheter, causing the expansion of the balloon.

This expansion of the balloon expands the artery, causing the destruction or dissolving of the clogging plaque.

The balloon is then completely deflated and the catheter is extracted.

In some cases a stent is used, that is, a wire mesh element in the shape of a cylinder fitted around the catheter balloon. During the expansion of the balloon this stent is deformed and enlarged. The balloon is deflated and the catheter withdrawn. The stent remains permanently in position, keeping the artery open and improving the blood flow.

Current angioplasty catheters present various drawbacks.

The section of catheter behind the balloon, that is the section of catheter opposite the inserting end, is relatively rigid. Consequently, during the expansion of the balloon, the portion of the catheter related to the balloon may undergo changes in position and angle.

The arteries on which action must be taken do not have a constant internal diameter, but they are often tapering, that is, they have a decreasing diameter. Consequently, the balloons of the known angioplasty catheters exert a greater pressure than necessary on the portion of the blood vessel with a smaller diameter, with the risk of dilating it excessively and/or tearing it.

To overcome all these drawbacks, a new angioplasty catheter has been designed and carried out.

### AIMS OF THE INVENTION

One of the aims of the new angioplasty catheter is to ensure greater flexibility between the portion fitted with the balloon and the remaining portion of the catheter.

Another aim of the new angioplasty catheter is to have a balloon with a truncated conical shape, so as to take adequate action in blood vessels with a decreasing internal diameter.

### DESCRIPTION OF THE INVENTION

These and other direct and complementary aims have been achieved through the development of a new angioplasty catheter comprising a tip portion, a proximal tube in flexible plastic material around which is placed the inflatable balloon, a multilayer distal tube in flexible plastic material, a metal tube for grip and connection.

On the proximal tube there are two marking radio-opaque metal rings.

It is possible to have also the proximal tube composed of two or more layers.

Angioplasty catheter having the tube portion inside the balloon composed of several layers of flexible plastic material overlapping and joined, and having the balloon in the shape of a truncated cone, once inflated.

### DESCRIPTION OF AN EMBODIMENT OF THE INVENTION

The characteristics of the new angioplasty catheter will be better clarified by the following description with reference to the drawings enclosed as an example without limitation.

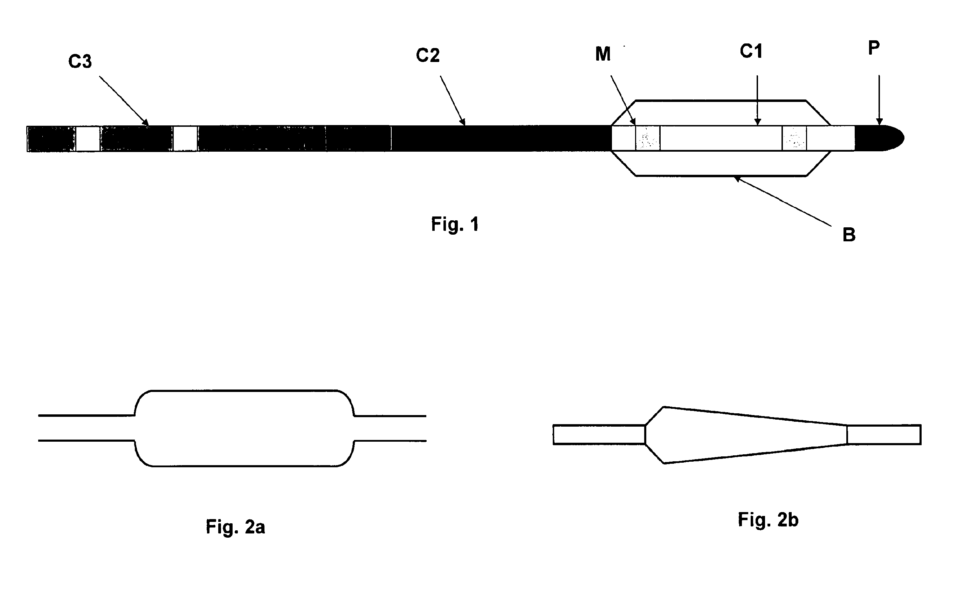

Figure 1 illustrates the new angioplasty catheter.

The new angioplasty catheter comprises a tip portion (P), a flexible proximal tube (C1) with radio-opaque markers (M) around which the inflatable balloon (B) is fitted, a flexible multilayer distal tube (C2), a metal tube (C3) for grip and connection.

The proximal tube (C1) is made of flexible and resistant plastic material. It is preferable that this proximal tube (C1) be made of PEBA.

On said proximal tube (C1) there are two radio-opaque markers (M) composed of two metal bands or rings, preferably platinum, with reduced thickness. Said markers (M) are applied to the proximal tube (C1) in specific positions, that is, close to the proximal and distal ends of the balloon (B). It is preferable that said markers (M) be applied to the proximal tube (C1) with cyanoacrylate adhesive specifically intended for medical use. The distal tube (C2) is made of resistant flexible multilayer plastic material. Typically it comprises three layers, the internal one of which is preferably made of PE-LM, the external layer is made of PEBA, the same material as the proximal tube (C1), while the joining intermediate layer is preferably made of Plexar.

The distal tube (C2) and the proximal tube (C1) are joined to each other with any method or procedure that ensures a perfect and resistant join, for example by laser welding. To facilitate the joining of the two tubes (C1, C2), the edge of the distal tube (C2) is enlarged and flared so as to overlap the edge of the proximal tube (C1).

The balloon (B) is made of elastic plastic material, that is expandable by means of air pressure or fluid at a relatively low pressure.

Said balloon (B) is preferably made of nylon, its shape has a diameter greater than that of the tubes (C1, C2), while the diameter at the ends is substantially the same as the external diameter of the tubes (C1, C2).

Said balloon (B), once inflated, may have a generally cylindrical shape, as shown in figure 2a, or a generically conical shape, as shown in figure 2b, with the smaller diameter facing the direction opposite the distal tube (C2).

The joined proximal tube (C1) and distal tube (C2) are inserted in said balloon (B) so as to position the balloon itself on the proximal tube (C1) in a central position with respect to the radio-opaque markers (M).

The ends of the balloon (B) are joined to the tube(s) (C1, C2) with any method or procedure that ensures a perfect and resistant join, for example by laser welding.

The tip portion (P) is constituted by an element in plastic material suited to be applied to the end of the proximal tube (C1) opposite the distal tube (C2), so as to fix the edge of the balloon (B) between said tip portion (P) and said proximal tube (C1).

The metal tube (C3) is fixed and joined to the end of the distal tube (C2) opposite the proximal tube (C1).

The new angioplasty catheter composed as described above presents considerable advantages.

The distal tube (C2) in multilayer plastic material allows greater flexibility than the portion of the catheter around the balloon (B).

The conical shape of the balloon (B) allows differentiated angioplasty to be obtained along the length of the blood vessel.

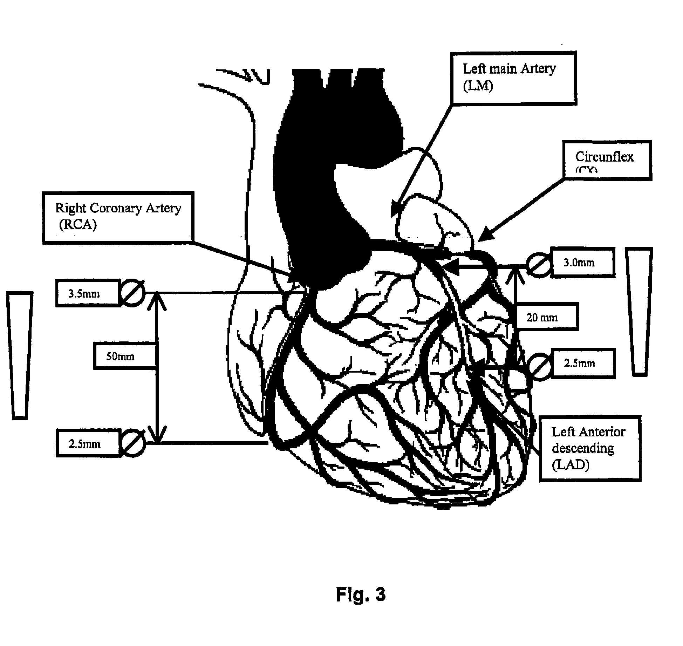

The conical shape of the balloon (B) also allows correct angioplasty to be performed in blood vessels with a decreasing diameter, for example the coronary arteries, as illustrated in figure 3.

Therefore with reference to the above description and to the enclosed figures, the following claims are made.

## Claims

1. Angioplasty catheter, **characterised in that** it comprises a proximal tube (C1) in flexible plastic material around which an inflatable balloon (B) is fitted, a tip portion (P) applied to one end of said proximal tube (C1), and wherein at the other end of the proximal tube (C1) there is a multilayer distal tube (C2) in flexible plastic material to which a metal tube (C3) is in turn applied in succession.

2. Angioplasty catheter according to claim 1, **characterised in that** the proximal tube (C1) is provided with at least two radio-opaque markers (M) located close to edges where the balloon (B) is joined to the proximal tube (C1).

3. Angioplasty catheter according to claim 2, **characterised in that** said radio-opaque markers (M) are made of platinum rings applied, glued or in some way fixed to said proximal tube (C1).

4. Angioplasty catheter according to previous claims, **characterised in that** the proximal tube (C1) is preferably made of PEBA.

5. Angioplasty catheter according to claims 1, 2, 3, 4, **characterised in that** the distal tube (C2) is made up of three of more layers of plastic material, and wherein the external layer is made of the same material as the proximal tube (C1).

6. Angioplasty catheter according to claim 5, **characterised in that** the internal layer is made of PE-LM.

7. Angioplasty catheter according to claim 5, **characterised in that** the intermediate layer is made of Plexar.

8. Angioplasty catheter according to claims 5, 6, 7, **characterised in that** the distal tube comprises other intermediate layers made of other and/or the same plastic materials.

9. Angioplasty catheter according to the previous claims, **characterised in that** the balloon (B), once inflated, assumes a generally cylindrical shape.

10. Angioplasty catheter according to claims 1, 2, 3, 4, 5, 6, 7, 8, **characterised in that** the balloon, once inflated, assumes a generically truncated conical shape.

11. Angioplasty catheter according to the previous claims, **characterised in that** the various tubes (C1, C2, C3) are joined and fixed together by means of laser welding.

12. Angioplasty catheter according to the previous claims, **characterised in that** it has a stent positioned on the balloon (B).
